# FASCICULE DE BREVET EUROPEEN

(11) **EP 0 402 208 B1**
(45) Date de publication et mention de la délivrance du brevet: **12.01.1994**
(21) Numéro de dépôt: 90401464.4
(22) Date de dépôt: 31.05.1990
(51) Int. Cl.: A61K 31/07, A61K 33/04, A61K 35/72

(54) **Association de vitamine A à dose physiologique et de différents principes actifs ayant une activité thérapeutique**
Mischung von Vitamin A in physiologischer Dosis und verschiedenen Wirkstoffen mit therapeutischer Wirksamkeit
Mixture of Vitamin A in physiological dosis and different active ingredients having therapeutic effect

(30) Priorité: 06.06.1989 FR 8907445
(43) Date de publication de la demande: 12.12.1990
(73) Titulaire: Grimberg, Georges Serge, F-75007 Paris (FR)
(72) Inventeur: Grimberg, Georges Serge, F-75007 Paris (FR)
(74) Mandataire: Madeuf, René Louis

(56) Documents cités:
- FR-A- 2 431 863
- UNLISTED DRUGS, vol. 27, no. 12, décembre 1975, Chatham, New Jersey, US

## Description

On connaît déjà des compositions thérapeutiques à base de vitamines A naturelles ou de vitamines A synthétiques auxquelles sont ajoutés en quantité suffisante du soufre minéral et organique, de la levure vivante et parfois un excipient véhiculaire pouvant être aromatisé.

Ceci est particulièrement le cas de médicaments décrits dans le FR - A - 2 228 470 mais les doses de cette composition pharmaceutique sont telles que la vitamine A est en trop grande proportion et peut être considérée comme un produit toxique car en dépassant les besoins journaliers, la vitamine A s'accumule dans l'organisme et dans certains cas provoque de graves perturbations.

Ainsi, l'utilisation de telles compositions pharmaceutiques est de plus en plus bloquée par les différents Ministères délivrant les autorisations de mise sur le marché des médicaments.

Au cours d'études très sérieuses, on a pu obtenir des résultats surprenants avec des doses faibles en vitamine A c'est-à-dire de l'ordre de 10 à 12 fois moins que dans les cas précédents.

De plus, on s'est aperçu également que la levure vivante employée ne l'était pas toujours à bon escient et, de ce fait, on a envisagé l'emploi de levure morte ne présentant pas les mêmes réactions secondaires donc mieux tolérée par l'organisme.

A la suite de ces études dont un compte rendu apparaîtra ci-après on a pu mettre au point un nouveau médicament thérapeutiquement actif destiné à remédier et à améliorer des désordres de la sphère ORL, de la dermatologie, de la rhumatologie, de la flore intestinale, et qui d'une manière plus générale a une activité immuno-stimulante, humorale et tissulaire.

Le nouveau médicament a donc pour résultat de donner à l'organisme la possibilité de se défendre lui-même du fait qu'il rétablit un équilibre rompu en particulier de rétablir le bon fonctionnement tissulaire des différents organes, de permettre la récupération de certaines fonctions déviées ou abolies, d'avoir une activité anti-infectieuse et ce malgré que les doses de vitamine A naturelles ou synthétiques soient très faibles. Ainsi le traitement peut être poursuivi pendant très longtemps en régularisant l'individu complètement sur un laps de temps suffisant.

Les indications thérapeutiques les plus indiquées de ce nouveau médicament sont : le catarrhe rhino-tubaire, l'otorrhée chronique, la surdité d'oreille interne, l'otospongiose opérée accompagnée d'un catarrhe tubaire, le glue otititis, l'amygdalite chronique, la rhinite purulente chronique, la sinusite, la rhino-pharyngite récidivante, l'acné, l'altération unguéale, la constipation, la diarrhée, la stimulation de l'immunité seule ou en association avec une vaccination.

Conformément à l'invention, l'association de vitamine A à dose physiologique, et de différents principes actifs ayant une activité thérapeutique tels que le soufre minéral et/ou organique associé ou non à des actifs biologiques tels que la levure morte, les lactobacilles, les germes gastro-intestinaux ou autres, morts ou atténués, est caractérisée en ce que la dose de vitamine A par gélule pour l'adulte est de 1700 U.I.

Suivant une particularité de l'invention, la dose de vitamine A par gélule pour le bébé est de 1000 U.I.

Suivant une seconde particularité de l'invention, la formule de la gélule pour l'adulte est de :
- Vitamine A 1700 U.I.
- L cystine base 72,6 mg
- Soufre sublimé lavé 22 mg
- Levure morte 77,4 mg

Suivant une troisième particularité de l'invention, la formule de la gélule pour le bébé est de :
- Vitamine A 1000 U.I.
- L cystine base 72,6 mg
- Soufre sublimé lavé 22 mg
- Levure morte 77,4 mg

Diverses autres caractéristiques de l'invention ressortent d'ailleurs de la description détaillée qui suit.

A titre d'information, il est indiqué qu'on peut, dans certains cas, utiliser une gélule dosée différemment et destinée soit à l'enfant dont l'âge est compris entre 30 mois et 15 ans soit à l'adulte c'est-à-dire que, dans certains cas, on trouve :
- Vitamine A 1500 U.I.
- L cystine base 72,6 mg
- Soufre sublimé lavé 22 mg
- Levure morte 77,4 mg

Pour obtenir une optique plus précise sur la tolérance thérapeutique de ces trois formules, on a été amené à faire une étude en double aveugle en utilisant la formule contre placebo pour 123 enfants à raison de deux gélules par jour pendant trois mois.

Il y a lieu de signaler que la formule active par gélule est de
- Vitamine A 1500 U.I.
- L cystine base 72,6 mg
- Soufre sublimé lavé 22 mg
- Levure morte 77,4 mg

Dans certains cas, il peut être nécessaire d'introduire en quantité variable du soufre minéral, du soufre organique (cystine ou autre), de l'actif biologique (levure morte, lactobacilles ou germes gastro-intestinaux ou autres germes morts et/ou atténués) en des quantités variables dans les formules en fonction d'une optique thérapeutique précise.

Au cours de la période hiverno-printanière 1987-1988, neuf médecins généralistes sous l'autorité d'une ORL pédiatre répartis sur l'ensemble du territoire français ont administré le nouveau médicament à des enfants venus consulter pour une infection de la sphère ORL. Ces enfants aux antécédents d'infections ORL récidivantes étaient parfaitement connus et suivis régulièrement par leur médecin.

Le traitement (nouveau médicament ou un placebo de même présentation galénique) était administré, en double aveugle, à la posologie de deux doses par jour pendant trois mois consécutifs.

Les enfants étaient revus lors de chaque infection ORL avec au minimum une consultation terminale en avril-mai pour effectuer le bilan de toute la période écoulée. Lors de chaque visite, le médecin notait le motif de la consultation avec le diagnostic correspondant, la prescription éventuelle d'un antibiotique, d'un repos au lit et le nombre de jours de repos.

123 enfants d'âge moyen de 4 ans et demi avec des extrêmes variant de deux à sept ans ont participé à l'étude. La durée de suivi, égale dans les deux groupes de traitement, a été en moyenne de dix sept semaines. Le nombre d'infections ORL est significativement plus faible chez les enfants traités par le nouveau médicament en comparaison aux enfants traités par le placebo. De même, le nombre de cures d'antibiotiques et la durée des absences pour un motif ORL sont significativement plus faibles dans le groupe du nouveau médicament par rapport au groupe placebo. Enfin, l'appréciation globale du médecin, quantifiée de O (résultat nul) à 3 (résultat très bon) est significativement meilleure pour le nouveau médicament. La tolérance a été excellente.

En conclusion, le nouveau médicament administré chez des enfants présentant une infection ORL diminue significativement les rechutes infectieuses ORL.

Les résultats sont nets entre les deux groupes, placébo/principes actifs. Le nombre d'infections ORL, le nombre de cures d'antibiotiques, le nombre de jours d'absences et leur durée sont nettement en faveur des "principes actifs".

Il est donc clairement établi, et cela pour la première fois, que cette formule bien que ne contenant qu'une posologie de Vitamine A couvrant les besoins dits physiologiques en Vitamine A et ne contenant pas de levure vivante mais de la levure morte a une action thérapeutique parfaitement établie.

En général, il est prévu une posologie qui est la suivante :
- Dose par jour pour un adulte: trois gélules
- Dose par jour pour un enfant: une ou deux gélules entre un mois et 15 ans en fonction de l'âge

## Revendications

1. Association de vitamine A et de différents principes actifs ayant une activité thérapeutique tels que le soufre minéral et/ou organique associé ou non à des actifs biologiques tels que la levure morte, les lactobacilles, les germes gastro-intestinaux ou autres, morts ou atténués, caractérisée en ce que la dose de vitamine A par gélule correspond à des doses physiologiques comprises entre 1000 U.I. et 1700 U.I.

2. Association suivant la revendication 1, caractérisée en ce que la dose de vitamine A par gélule pour l'adulte est au maximum de 1700 U.I..

3. Association suivant la revendication 1, caractérisée en ce que la dose de vitamine A par gélule pour le bébé est de 1000 U.I.

4. Association suivant la revendication 2, caractérisée en ce que la formule de la gélule pour l'adulte est de :
- Vitamine A 1700 U.I.
- L cystine base 72,6 mg
- Soufre sublimé lavé 22 mg
- Levure morte 77,4 mg

5. Association suivant la revendication 3, caractérisée en ce que la formule de la gélule pour le bébé est de :
- Vitamine A 1000 U.I.
- L cystine base 72,6 mg
- Soufre sublimé lavé 22 mg
- Levure morte 77,4 mg

6. Association suivant les revendications 1, 2 et 4 caractérisée en ce que la formule de la gélule pour l'enfant et l'adulte est de :
- Vitamine A 1500 U.I.
- L cystine base 72,6 mg
- Soufre sublimé lavé 22 mg
- Levure morte 77,4 mg

7. Association suivant l'une des revendications 1 à 6, caractérisée en ce que le soufre minéral, le soufre organique (cystine ou autre), l'actif biologique (levure morte, lactobacilles ou germes gastro-intestinaux ou autres germes morts et/ou atténués) ont des quantités variables dans les formules en fonction d'une optique thérapeutique précise.

## Patentansprüche

1. Mischung von Vitamin A und verschiedenen Wirkstoffen mit einer therapeutischen Wirksamkeit in der Art des mineralischen und/oder organischen Schwefels in oder ohne Mischung mit biologischen Wirkstoffen wie z.B. der abgetöteten Hefe, den Laktobazillen, den gastrointestinalen Keimen oder anderen, abgetöteten oder abgeschwächten, **dadurch gekennzeichnet,** daß die Dosis des A-Vitamins pro Kapsel physiologischen Dosen zwischen 1.000 I.E. und 1700 I.E. entspricht.

2. Mischung nach Anspruch 1, **dadurch gekennzeichnet,** daß die Dosis des A-Vitamins pro Kapsel für den Erwachsenen 1.700 I.E. beträgt.

3. Mischung nach Anspruch 1, **dadurch gekennzeichnet,** daß die Dosis des A-Vitamins pro Kapsel für das Kleinkind 1.000 I.E. beträgt.

4. Mischung nach Anspruch 2, **dadurch gekennzeichnet,** daß die Formel für die Kapsel für den Erwachsenen wie folgt lautet:
- Vitamin A 1700 I.E.
- Basis-L-Cystin 72,6 mg
- gewaschener sublimierter Schwefel 22 mg
- abgetötete Hefe 77,4 mg

5. Mischung nach Anspruch 3, **dadurch gekennzeichnet,** daß die Formel für die Kapsel für das Kleinkind wie folgt lautet:
- Vitamin A 1.000 I.E.
- Basis-L-Cystin 72,6 mg
- gewaschener sublimierter Schwefel 22 mg
- abgetötete Hefe 77,4 mg

6. Mischung nach den Ansprüchen 1 bis 5, **dadurch g**e**kenn****zeichnet,** daß die Formel für die Kapsel für das Kind und den Erwachsenen wie folgt lautet:
- Vitamin A 1.500 I.E.
- Basis-L-Cystin 72,6 mg
- gewaschener sublimierter Schwefel 22 mg
- abgetötete Hefe 77,4 mg

7. Mischung nach einem der Ansprüche 1 bis 6, **dadurch** **gekennzeichnet,** daß der mineralische Schwefel, der organische Schwefel (Cystin oder anderer), der biologische Wirkstoff (abgetötete Hefe, Laktobazillen oder gastrointestinale Keime oder andere abgetötete und/oder abgeschwächte Keime) veränderliche Mengen in den Formeln aufweisen und zwar in Abhängigkeit eines therapeutischen Gesamtbildes.

## Claims

1. Association of vitamin A and of various active principles having a therapeutical activity such as mineral and/or organic sulphur, associated or not with biologically active principles such as dead yeast, lactobacilli, gastrointestinal microorganisms or others, either dead or attenuated, characterized in that the quantity of vitamin A per capsule corresponds to physiological quantities comprised between 1000 I.U. and 1700 I.U.

2. Association according to claim 1, characterized in that the quantity of vitamin A per capsule for an adult is at most 1700 I.U.

3. Association according to claim 1, characterized in that the quantity of vitamin A per capsule for a baby is 1000 I.U.

4. Association according to claim 2, characterized in that the formula of a capsule for an adult is :
- Vitamin A 1700 I.U.
- Base L cystine 72.6 mg
- Washed sublimed sulphur 22 mg
- Dead yeast 77.4 mg

5. Association according to claim 3, characterized in that the formula of a capsule for a baby is :
- Vitamin A 1000 I.U.
- Base L cystine 72.6 mg
- Washed sublimed sulphur 22 mg
- Dead yeast 77.4 mg

6. Association according to claims 1, 2 and 4, characterized in that the formula of a capsule for a child and an adult is :
- Vitamin A 1500 I.U.
- Base L cystine 72.6 mg
- Washed sublimed sulphur 22 mg
- Dead yeast 77.4 mg

7. Association according to one of claims 1 to 6, characterized in that the mineral sulphur, the organic sulphur (cystine or other), the biologically active principles (dead yeast, lactobacilli or gastrointestinal microorganisms or other dead and/or attenuated microorganisms) are in variable quantities in the formulas as a function of an accurate therapeutical action.
